# EUROPEAN PATENT APPLICATION

(11) **EP 3 480 825 A1**
(43) Date of publication of application: **08.05.2019**
(21) Application number: 17199813.1
(22) Date of filing: 03.11.2017
(51) Int. Cl.: G16H 50/70, G16H 50/20, A61B 5/11, A61B 5/00

(54) **CLASSIFYING A DISEASE OR DISABILITY OF A SUBJECT**

(71) Applicant: Koninklijke Philips N.V., 5656 AE Eindhoven (NL)
(72) Inventor: DAERR, Heiner, 5656 AE Eindhoven (NL); EWALD, Arne, 5656 AE Eindhoven (NL)
(74) Representative: de Haan, Poul Erik

(57) **Abstract**

Presented are concepts for m classifying a disease or disability of a subject. One such concept comprises obtaining interaction data associated with a subject, the interaction data being representative of the subject's interaction with a movement-based input device. The interaction data is processed with a first machine learning process to determine a set of characteristics for describing the subject. The set of characteristics is then processed with a second machine-learning process to generate a classification result for the subject.

## Description

### FIELD OF THE INVENTION

This invention relates to the field of disease or disability assessment and more particularly to classifying a state or progression of a disease or disability of a subject (such as a patient).

### BACKGROUND OF THE INVENTION

The assessment of a state or progression of a disease or disability of a subject is a widely known problem.

It is known that many diseases or disabilities have associated and/or characteristic movement disorders (which may, for instance, become more severe as the disease or disability progresses). For example, Parkinson's disease (PD) has four motoric symptoms that are considered to be cardinal: tremor, rigidity, slowness of movement, and postural instability. Similarly, other diseases involving mental and/or physical disorders may exhibit different motoric symptoms. Such motoric systems are known to impact how a subject interacts with a movement-based input device (such as a mouse, pointing device, track ball, etc.) when compared to a healthy person.

It has been previously proposed that motoric symptoms exhibited by a subject may be used to identify a disease and/or its state. For example, previous work has investigated the feasibility of using the features and methodology of a spirography application (originally designed to detect early PD motoric symptoms) for automatically assessing motor symptoms of advanced PD patients experiencing motor fluctuations. Such work combined spirography data and clinical assessments as assessed by a clinician who observed animated spirals in a web interface. Accordingly, a visualisation technique was proposed which presented visual clues to clinicians as to which parts of a spiral drawing (or its animation) may be important for the given classification. This has the drawback that it relies on a clinician or medical professional to observe how a subject draws a spiral. Also, such observation and/or assessment may be highly subjective and rely on high levels of expertise.

Thus, there remains a need for an efficient and/or effective approach to assessing a state or progression of a disease or disability of a subject.

### SUMMARY OF THE INVENTION

The invention aims to at least partly fulfil the aforementioned needs. To this end, the invention provides devices, systems and methods as defined in the independent claims. The dependent claims provide advantageous embodiments.

There is provided a method for classifying a disease or disability of a subject. The method comprises obtaining interaction data associated with a subject, wherein the interaction data is representative of the subject's interaction with a movement-based input device. The method further comprises processing the interaction data with a first machine learning process to determine a set of characteristics for describing the subject. The method also comprises processing the set of characteristics with a second machine-learning process to generate a classification result for the subject. The classification result is representative of a state or progression of a disease or disability of the subject.

Proposed is a concept of analysing a subject's interaction with a movement-based interaction device (such as a mouse, pointing device, or the like) using a machine learning process so as to characterise the subject. This characterising of the subject can then be analysed with a second machine learning process to classify the subject.

Unlike conventional approaches of assessing a disease or disability of a subject which require specialist knowledge and expertise of a medical professional, proposed embodiments take a contrary approach of analysing device interactions with machine learning processes to assess behavioural biometrics and identify characteristic symptoms of a disease or disability. Further, identified symptoms may be analysed to assess a stage and/or progression of the disease or disability.

A concept is thus proposed which may analyse individual behavioural biometrics from a subject's interaction with an input device. The device might, for example, comprise all or part of a computer, smartphone, tablet computer, portable computing device, or the like. For instance, the movements of a mouse device may be characterised for a subject and such characteristics may then be used for identification and/or assessment of a disease or disability.

Because diagnosis or assessment of diseases (like Parkinson's disease or Alzheimer's disease for example) or disabilities (like acquired brain injury or physical disability) is typically difficult, proposed embodiments may be beneficial due to providing new or additional information that may facilitate improved (e.g. simpler or more accurate) disease identification, assessment and/or management. For example, embodiments may be useful for monitoring a course of medication and/or therapy for a disease or disability. They may, for instance, provide objective measures for identifying if a drug and/or therapy improves or alleviates motoric symptoms of a subject.

Thus, there is proposed an approach to analysing data relating to a subject's interaction with an input device using machine learning processes. Such analysis result(s) may enable a disease or disability of a subject to be identified and/or classified into one of a plurality of stages. Such a proposed approach includes analysing detected movement of an input device using a first machine learning process so as to identify a set of characteristics of the subject. The set of characteristics can then be analysed using a second machine learning process to classify the characteristics to one of a plurality of classed. Classification of the subject characteristics may be performed using a machine learning process that has been trained with known disease characteristics and classes, thereby enabling automatic classification of a subject with respect to a disease. In this way, classification can be performed in a supervised machine learning process which exploits data of subject with known disease states.

Embodiments may therefore enable a subject to be automatically and accurately matched to a disease or disability state based on interaction with a movement-based input device. Further, repeated classification over time may enable the monitoring or tracking of a subject's disease or disability, thus enabling assessment of medication and/or therapy effectiveness. Also, therapy or medication decisions based on an earlier classification process may be monitored and/or detected so as to provide feedback information which can be used to refine or improve subsequent classification processes.

In particular, embodiments may be used in relation to a subject (e.g. a patient) and medication or therapy so as optimize implementation or allocation of the medication or therapy for the subject. Such embodiments may support clinical planning. Improved Clinical Decision Support (CDS) may therefore be provided by proposed concepts.

Also, the collection and analysis of data relating to a subject's use of a movement-based input device may facilitate correlation of subject and disease-specific characteristics, which may, in turn, be used for subject-specific or tailored diagnostics. Proposed approaches may focus on the combination of data relating to a subject and data relating to interaction with an input device to enable efficient and flexible disease classification. By way of example, this may provide for: reduced subject administration or interrogation; improved disease or disability management; creation of best practice diagnostic procedures; and iterative improvement of subject/disease-specific diagnostics, treatment and management.

For instance, a subject's interaction with an input device may be recorded during use of an application (e.g. word processing application, or web browser), for example via software running in the background. Alternatively, a specifically-designed computer program, like a computer game, may be provided which provide instructions or challenges to the subject and then the user's use of an input device in response to the instructions/challenges may be recorded to measure behavioural biometrics. The device interactions (e.g. movements of the input device) may then be provided to an artificial neural network which is adapted to identify characteristics of the subject, and then these characteristics may be provided to another artificial neural network which is adapted to compare the characteristics with known characteristics of one or more diseases or disabilities, thereby identifying characteristic symptoms of a disease or disability, staging symptoms or comparing symptoms to previous sessions.

It will therefore be appreciated that proposed embodiments may enable the monitoring and staging of diseases or disabilities involving motor symptoms or mental disorders.

Processing the set of characteristics with the second machine-learning process may comprise: comparing the set of characteristics with classification data representing one or more associations between characteristics and disease states; and based on the result of the comparison, applying a machine-learning based classification process to the set of characteristics. Such embodiments may therefore work in a supervised fashion by exploiting data relating to patients with known diseases and/or disease states. The second machine-learning process may thus be trained to identify a specific user and/or specific disease or disability state, thereby enabling more accurate results to be obtained whilst minimising human involvement.

The classification result for the subject may comprise an identification of class or value within a predetermined range of available classes or values for the disease, and optionally wherein the identification comprises a numerical value. For example, the identification may comprise a text value, such as written description of a disease or disease state, or a numerical value, such as a number between 0-1 or 1%-100% for example. In this way, a disease or disease state of a subject (such as a "degree of patient disease" for example) may be classified and represented using an identifier that is easy to understand and/or or simple to implement in conjunction with a classification process.

Proposed embodiments may further comprise the step of providing an instruction to the subject for directing the subject to interact with the movement-based input device. Embodiments may therefore be adapted to obtain specific or preferred data relating to a subject's motor symptom(s). In this way, more relevant and/or accurate data representative of a subject's motor symptom(s) may be obtained. The provision of useless and/or irrelevant data may thus be avoided, thereby alleviating processing and/or storage requirements.

For example, the instruction may define a challenge, and the challenge may comprise a time-varying parameter. A game or interactive application may therefore be provided which is adapted to set specific challenges to a subject, which, in turn, then enables information of particular interest to be obtained (such as behavioural biometrics).

In an embodiment, at least one of the first machine learning process and second machine learning process may employ an artificial neural network. Proposed embodiments may therefore implement known artificial intelligence architectures which can serve as components or building blocks for implementing required processes.

For example, the step of processing the interaction data with a first machine learning process may comprise processing the interaction data with an artificial neural network, and then step of processing the set of characteristics with a second machine-learning process may comprise processing the set of characteristics with a second artificial neural network. The machine learning processes may thus be sequentially employed.

In an embodiment, the method may further comprise: training the first machine learning process with interaction data representative of a training subject's interaction with a movement-based input device; and training the second machine learning process with: classification data representative of a state of a disease or disability of the training subject; and data generated from training the first machine learning process.

Proposed embodiments may further comprise the step of assessing at least one of a medication program and treatment program for the subject based on the classification results. Such embodiments may, for example, enabling assessment of medication and/or therapy effectiveness. This may be beneficial for the monitoring or tracking of a subject's disease or disability. Also, therapy or medication decisions based on an earlier classification process may be monitored and/or detected so as to provide feedback information which can be used to refine or improve subsequent classification processes. Thus, an iterative process of refining the classification process may be employed using observations in response to a medication program or treatment program.

The subject may be a patient. Embodiments may therefore be of particular benefit for medical or clinical applications where the diagnosis of patients and/or the allocation of medical/clinical treatment may be important. For instance, proposed embodiments may help to define a treatment program that is tailored or optimized for a specific individual or subject. This may, for example, provide numerous benefits including: reduction in subject administration and repetitive interrogation overhead; improvement in subject comfort, increased resource throughput, usage and quality; facilitate the creation of consistent best practice for diagnosis processes; and enable iterative improvement of subject/disease specific resource usage.

According to another aspect of the invention, there may be provided a computer program product for classifying a disease or disability of a subject, wherein the computer program product comprises a computer-readable storage medium having computer-readable program code embodied therewith, the computer-readable program code configured to perform all of the steps of a method according to a proposed embodiment.

According to another aspect of the invention, there is provided a system for classifying a state of a disease or disability of a subject. The system comprises: an input interface adapted to obtain interaction data associated with a subject, the interaction data being representative of the subject's interaction with a movement-based input device; a first machine learning unit adapted to process the interaction data with a first machine learning process to determine a set of characteristics for describing the subject; and a second machine learning unit adapted to process the set of characteristics with a second machine-learning process to generate a classification result for the subject, the classification result being representative of a state or progression of a disease or disability of the subject.

The system further comprise an output interface adapted to provide an instruction to the subject for directing the subject to interact with the movement-based input device. For example, the instruction may define a challenge, and the challenge may comprise a time-varying parameter.

The system may further comprise an analysis unit adapted to assess at least one of a medication program and treatment program for the subject based on the classification results and to generate an output signal based on the result of the assessment.

The one or more components of the system may be remotely located from a display unit, and a control signal may thus be communicated to the display unit via a communication link. In this way, a user (such as a resource administrator) may have an appropriately arranged display system that can receive and display information at a location remotely located from one or more components of the system. Embodiments may therefore enable a user to remotely review information (e.g. a classification result, instruction, assessment of medication or treatment program, etc.) generated by the system using a portable display device, such as a laptop, tablet computer, mobile phone, PDA, etc.

The system may further comprise: a server device comprising the machine learning units; and a client device comprising the display unit. Dedicated data processing means may therefore be employed for the purpose of generating a classification result for the subject, thus reducing processing requirements or capabilities of other components or devices of the system.

The system may further comprise a client device, wherein the client device comprises at least one of the machine learning units and the display unit. In other words, a user (such as a medication administrator or medical professional) may have an appropriately arranged client device (such as a laptop, tablet computer, mobile phone, PDA, etc.) which processes received data in order to generate classification result for the subject and generate a control signal.

Thus, it will be understood that processing capabilities may therefore be distributed throughout the system in different ways according to predetermined constraints and/or availability of processing resources.

According to another aspect of the invention, there is provided a device for classifying a state of a disease or disability of a subject, the device comprising: an input interface adapted to obtain interaction data associated with a subject, the interaction data being representative of the subject's interaction with a movement-based input device; a first machine learning unit (220) adapted to process the interaction data with a first machine learning process to determine a set of characteristics for describing the subject; and a second machine learning unit (250) adapted to process the set of characteristics with a second machine-learning process to generate a classification result for the subject, the classification result being representative of a state or progression of a disease or disability of the subject.

These and other aspects of the invention will be apparent from and elucidated with reference to the embodiment(s) described hereinafter.

### BRIEF DESCRIPTION OF THE DRAWINGS

Examples in accordance with aspects of the invention will now be described in detail with reference to the accompanying schematic drawings, in which:
Figure 1 is an exemplary flow diagram of a method for classifying a disease or disability of a subject according to an embodiment;
Figure 2 is an illustration summarising a training process for a system according to a proposed embodiment;
Figure 3 is an illustration summarising an application of a trained system according to a proposed embodiment;
Figure 4 is a simplified block diagram of a system according to an embodiment; and
Figure 5 is a simplified block diagram of a computer within which one or more parts of an embodiment may be employed.

### DETAILED DESCRIPTION OF THE EMBODIMENTS

Proposed is a concept for classifying a disease or disability of a subject. Data representative of the subject's interaction with a movement-based input device may be obtained and processed using a machine learning process (such as that of an artificial neural network) to generate a classification result for the subject. The classification result may, for instance, be representative of a state or progression of a disease or disability of the subject. By employing machine learning processes, a subject's use of an input device may be characterised (by a first unsupervised machine learning process) and then classified (by a second supervised machine learning process) so as to facilitate automated analysis of behavioural biometrics using existing knowledge that has been used to train or supervise the machine learning process(es). Proposed embodiments employ a concept of using data about movement of an input device manipulated by a subject to extract motoric symptoms of the subject.

Embodiments may thus be useful for identifying a disease or disability (or stage thereof) suffered by a subject (such as a patient or individual) so that resource therapy or medication can be tailored to the subject. For instance, where the subject is a patient, proposed concepts may be useful for assessing the progression of a disease or disability. Decisions regarding suitability of prescribed therapy and/or medication program may thus be assisted by proposed embodiments. Improved clinical planning and therapy may therefore be provided according to specific characteristics of individual subjects.

Embodiments of the present invention are therefore directed toward enabling subject-specific treatment so as to facilitate or enhance a CDS process. Further, embodiments may be aimed at enabling the provision of subject-specific therapy or treatment that make use of medication and/or resources in an optimal manner.

Embodiments are based on the insight that machine learning processes may be employed to analyse data relating to movement characteristics of a subject so as to identify a disease state or progression. Artificial intelligence components (such as artificial neural networks or recurrent neural networks) may therefore be leveraged in conjunction with movement tracking of a subject so as to classify a disease state of the subject.

By way of example only, illustrative embodiments may be utilized in many different types of clinical, medical or patient-related environments, such as a hospital, doctor's office, ward, care home, person's home, etc. In order to provide a context for the description of elements and functionality of the illustrative embodiments, the Figures are provided hereafter as examples of how aspects of the illustrative embodiments may be implemented. However, it should be appreciated the Figures are only examples and are not intended to assert or imply any limitation with regard to the environments, systems or methods in which aspects or embodiments of the present invention may be implemented. For example, embodiments may not be limited to matching a subject to medical resources, but may instead be used in conjunction with other types or forms of subjects and resources.

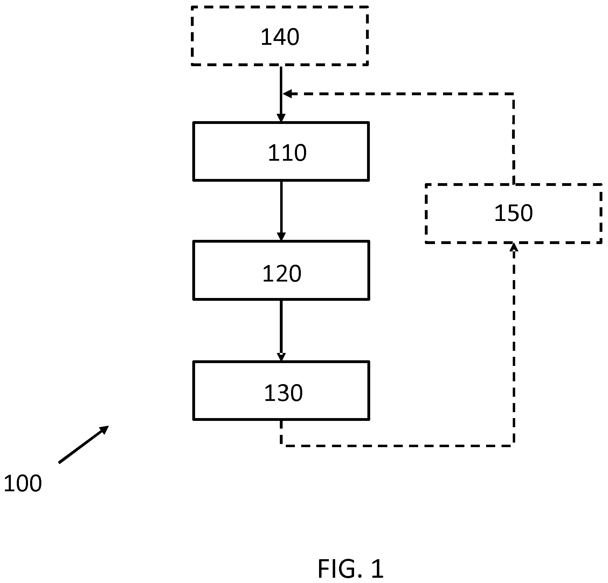

Referring now to Figure 1, there is depicted an exemplary flow diagram of a method 100 for classifying a disease or disability of a subject according to an embodiment. Here, the subject (i.e. a person receiving or registered to receive medical treatment) is known to be suffering from PD.

The method begins in step 110 wherein interaction data associated with the subject is obtained. The interaction data is representative of the subject's interaction with a movement-based input device. More specifically, the interaction data comprises data representative of the movements of a pointing device (such as a mouse device) in the x-direction and y-direction as a function of time. Thus, interaction data is a function of x-position, y-position, and time, i.e. f(x,y,t), and the data may thus be regarded as a two-dimensional time series. In this example, the subject uses the pointing device to control the position of a cursor displayed on a screen and is instructed to try to follow a moving point also displayed on the screen (wherein the point changes its direction and speed of movement rapidly and/or randomly).

Next, in step 120, the obtained interaction data is processed by a first, unsupervised machine learning process to determine a set of characteristics for describing the subject. Here, the first machine learning process is implemented using an artificial neural network that has previously been trained using interaction data associated with subjects having known characteristics.

The method then proceeds to step 130 which comprises processing the set of characteristics with a second, supervised machine-learning process to generate a classification result for the subject. More specifically, the step 130 of processing the set of characteristics with a second, supervised machine-learning process comprises: comparing the set of characteristics with classification data representing one or more associations between characteristics and disease states; and based on the result of the comparison, applying a machine-learning based classification process to the set of characteristics.

Here, a classification result comprises an identification of a state or progression of a disease or disability of the subject. By way of example, the identification of class or value comprises a numerical value, such as a number between 0-1 or 1%-100%. It will therefore be understood that the identification of class or value is adapted to represent or describe a disease (or state of a disease) of the subject, and may thus comprise a numerical value, alphanumerical value, text value, colour, and other suitable identifier of value.

Thus, completion of step 130 generates a classification value for the subject. For instance, the classification value for the subject may be degree of severity of PD for the subject and may comprise a value between 0 and 1, wherein 1 represents a highest level of severity and 0 represents a lowest level of severity. In this way, a disease or disability of the subject can be classified and represented using an identifier that is easy to understand and/or or simple to implement in conjunction with a decision process.

Further to the method described above, it is noted that embodiments may actually include comprise the step 140 of providing an instruction to the subject for directing the subject to interact with the movement-based input device. For example, the instruction may define a challenge (such as a game, task or test) that has a time-varying parameter in order to require the subject to change, adapt or modify movement of the input device as time elapses. Such an additional step is depicted in Figure 1 by the dashed box labelled "140".

Furthermore, after completion of step 130, prescribed therapy and/or medication (e.g. that prescribed based on the classification process of steps 110 through 130) may be monitored and/or detected so as to provide feedback information which can be used to refine or improve subsequent classification processes. Such an additional process is depicted in Figure 1 by the dashed box labelled "150" and it associated dashed arrows. In this way, embodiments may include a process of assessing a medication and/or treatment program based on the classification results, and then feedback may be used to modify or refine the classification process for improved accuracy and/or efficiency of classification. Thus, an iterative process of refining the classification processes may be employed.

It will be appreciated that the machine learning processes employed by proposed embodiments may be trained by subjects with known characteristics and/or disease states. For instance, for training of an artificial neural network used by an embodiment, a computer game can be played by a healthy subject (or a subject with a known status of a disease) and the recorded movements of the input device used by the subject then provided to the artificial neural network.

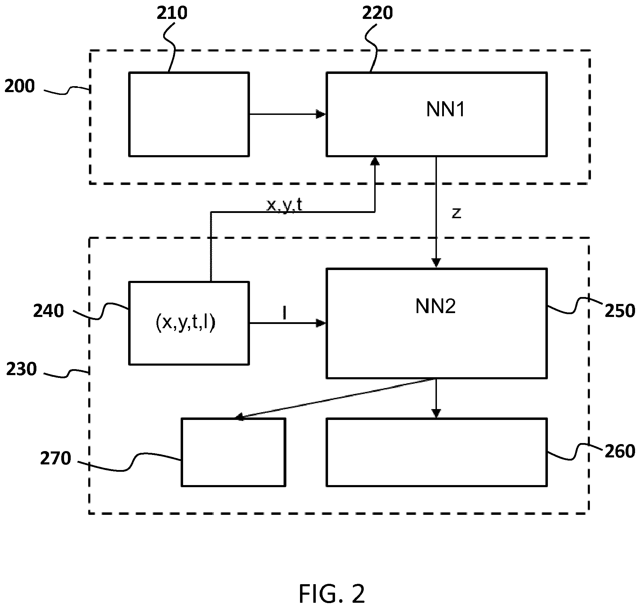

Figure 2 illustrates a concept of training first and second machine learning process employed by an embodiment. In a first training phase 200, raw, unlabelled input data 210, i.e. f(x,y,t) captured from a movement-based input device is used to train a first Artificial Neural Network (ANN) 220 ("NN1") in an unsupervised fashion. Accordingly, the data 210 is 'unlabelled', meaning that no disease state is known and just the raw input device data (e.g. x-position on monitor, y-position on monitor and time t) is provided. Here, the first ANN 220 is adapted to learn specific features of the input data 210 by mapping the input data 210, i.e. f(x,y,t), to a lower dimensional vector representation z from which the original data could in principle be reconstructed. More specifically, the features identified are set 'z' of characterising features that may be regarded as the subject's fingerprint.

In a second training phase 230, a second ANN 250 ("NN2") is provided with the output z of the first ANN 220, i.e. the subject's fingerprint 'z'. The second ANN 250 is also provided with (labelled) data '1' of patients with known disease states, which thus acts as supervisory data for supervising the learning of the second ANN 250. The training is undertaken in a supervised fashion by exploiting data of patients with known disease state labelled as '1'. In the second training phase labelled data 1 is used to train the second ANN 250 based on the label and the output z of the first ANN 220 so as to identify a subject's disease state 260 and/or a specific subject 270.

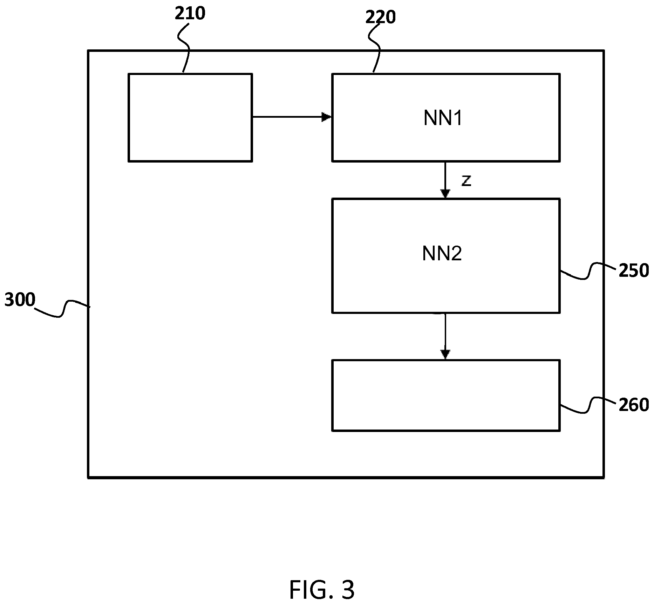

Referring now Figure 3, there is illustrated an example embodiment of a system 300 that has completed the training process depicted in Figure 2.

Unlabelled input data 210, i.e. f(x,y,t) captured from a movement-based input device is provided to the first ANN 220 (NN1). The first ANN 220 is adapted to perform a first machine learning process so as to map the input data 210 to a set z of characterising features that may be regarded as the subject's fingerprint. The set z of characterising features is then provided to the second ANN 250 (NN2) which is adapted to perform a second machine learning process so as to identify the subject's disease state 260. Thus, in such an application scenario, the data from the movement-based input device is analyzed by the previously trained neural networks in order to identify a particular disease state of the subject.

Further, by comparing the identified disease state with previously identified states, a progression of the subject's disease may also be assessed.

It will be understood that proposed AI employed by the proposed machine learning processes may be built upon conventional or widely known AI architectures. Accordingly, detailed discussion of specific AI implementations is omitted for the sake of brevity and/or clarity of the proposed concepts detailed herein. Nonetheless, purely by way of example and completeness, it is noted that proposed embodiments may employ recurrent neural networks (RNNs). A first type of such a RNN may be a simple recurrent network (SRN) which models temporal trajectories of a data sequence to infer an unknown future observation. A second type of such a RNN may be a long short-term memory (LSTM) that enables modelling of longer trajectories by exploiting forgetting switches. Non-linear, temporal evolution of medial status of human subjects may be approximated by such RNNs and the prediction of future measurements may be more accurate than those of a linear approximation method.

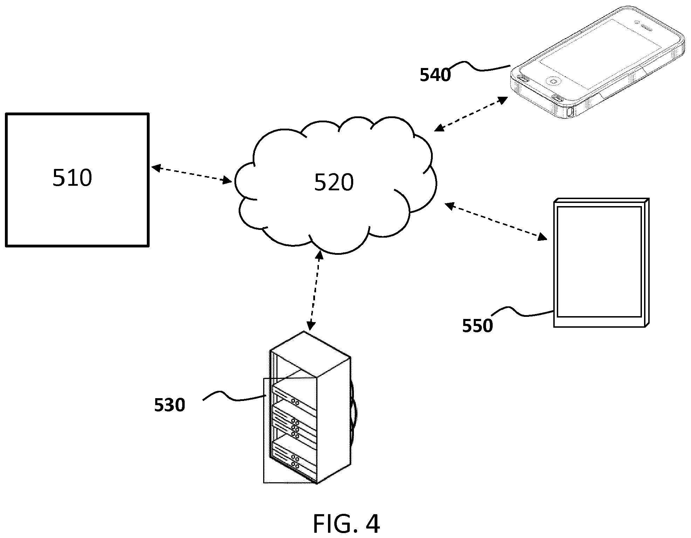

Referring now to Fig. 4, there is depicted an embodiment of a system according to an embodiment of the invention comprising an input system 510 arranged to obtain interaction data associated with a subject.

Here, the input system 510 is adapted to obtain interaction data associated with a subject using a pointing device, the obtained interaction data being representative of the subject's interaction with the pointing device. The input system 510 is adapted to output one or more signals which are representative of obtained interaction data.

The input system 510 communicates the output signals via a network 520 (using a wired or wireless connection for example) to a remotely-located data processing system 530 (such as server).

The data processing system 530 is adapted to receive the one or more output signals from the input system 510 and process the received signal(s) with a first machine learning process to determine a set of characteristics for describing the subject. The data processing system 530 is also adapted to process the set of characteristics with a second machine-learning process to generate a classification result for the subject. The classification result is representative of a state or progression of a disease or disability of the subject. Thus, the data processing 530 provides a centrally accessible AI processing resource that can receive information from the input system 510 and run one or more AI algorithms to transform the received information into a classification result that is representative of a state or progression of a disease or disability of the subject. Information relating to the classification result can be stored by the data processing system (for example, in a database) and provided to other components of the system. Such provision of information about a subject's classification may be undertaken in response to a receiving a request (via the network 520 for example) and/or may be undertaken without request (i.e. 'pushed').

For the purpose of receiving information about a subject's classification from the data processing system 530, and thus to enable subject-specific information to be viewed, the system further comprises first 540 and second 550 mobile computing devices.

Here, the first mobile computing device 540 is a mobile telephone device (such as a smartphone) with a display for displaying information in accordance with embodiments of the proposed concepts. The second mobile computing device 550 is a mobile computer such as a Laptop or Tablet computer with a display for displaying information in accordance with embodiments of the proposed concepts.

The data processing system 530 is adapted to communicate classification output signals to the first 540 and second 550 mobile computing devices via the network 520 (using a wired or wireless connection for example). As mentioned above, this may be undertaken in response to receiving a request from the first 540 or second 550 mobile computing devices.

Based on the received output signals, the first 540 and second 550 mobile computing devices are adapted to display one or more graphical elements in a display area provided by their respective display. For this purpose, the first 540 and second 550 mobile computing devices each comprise a software application for processing, decrypting and/or interpreting received output signals in order to determine how to display graphical elements. Thus, the first 540 and second 550 mobile computing devices each comprise a processing arrangement adapted to determine an attribute of a subject-specific classification, and to generate a display control signal for modifying at least one of the size, shape, position, orientation, pulsation or colour of a graphical element based on the determined classification of the subject.

The system can therefore communicate information about subject's disease state or progression to users of the first 540 and second 550 mobile computing devices. For example, each of the first 540 and second 550 mobile computing devices may be used to display graphical elements to a medical practitioner, doctor, consultant, technician or caregiver for example.

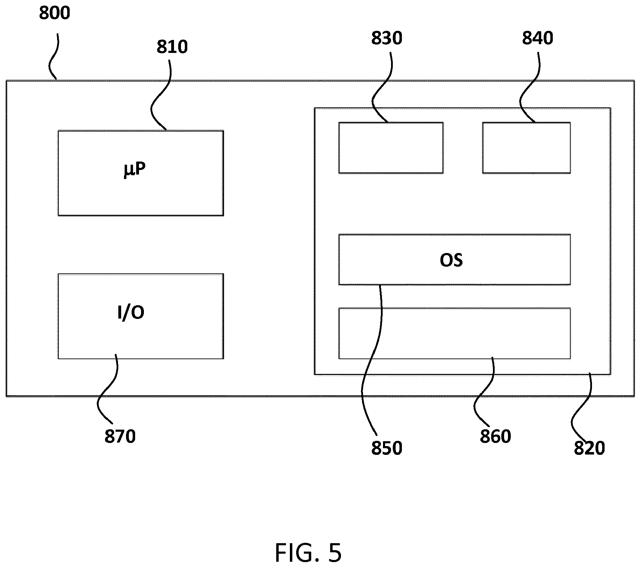

Implementations of the system of Figure 5 may vary between: (i) a situation where the data processing system 530 communicates display-ready data, which may for example comprise display data including graphical elements (e.g. in JPEG or other image formats) that are simply displayed to a user of a mobile computing device using conventional image or webpage display (can be web based browser etc.); to (ii) a situation where the data processing system 530 communicates raw data set information that the receiving mobile computing device then processes with first and second machine learning processes to generate a classification result, and then displays graphical elements based on the determined classification result (for example, using local software running on the mobile computing device). Of course, in other implementations, the processing may be shared between the data processing system 530 and a receiving mobile computing device such that data groups generated at data processing system 530 is sent to the mobile computing device for further processing by local dedicated software of the mobile computing device. Embodiments may therefore employ server-side processing, client-side processing, or any combination thereof.

Further, where the data processing system 530 does not 'push' information about a subject-specific disease state or progression, but rather communicates information in response to receiving a request, the user of a device making such a request may be required to confirm or authenticate their identity and/or security credentials in order for information to be communicated.

Figure 5 illustrates an example of a computer 800 within which one or more parts of an embodiment may be employed. Various operations discussed above may utilize the capabilities of the computer 800. For example, one or more parts of a system for classifying a disease or disability of a subject may be incorporated in any element, module, application, and/or component discussed herein.

The computer 800 includes, but is not limited to, PCs, workstations, laptops, PDAs, palm devices, servers, storages, and the like. Generally, in terms of hardware architecture, the computer 800 may include one or more processors 810, memory 820, and one or more I/O devices 870 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the computer 800, and the processor 810 may be a semiconductor based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 820 can have a distributed architecture, where various components are situated remote from one another, but can be accessed by the processor 810.

The software in the memory 820 may include one or more separate programs, each of which comprises an ordered listing of executable instructions for implementing logical functions. The software in the memory 820 includes a suitable operating system (O/S) 850, compiler 840, source code 830, and one or more applications 860 in accordance with exemplary embodiments. As illustrated, the application 860 comprises numerous functional components for implementing the features and operations of the exemplary embodiments. The application 860 of the computer 800 may represent various applications, computational units, logic, functional units, processes, operations, virtual entities, and/or modules in accordance with exemplary embodiments, but the application 860 is not meant to be a limitation.

The operating system 850 controls the execution of other computer programs, and provides scheduling, input-output control, file and data management, memory management, and communication control and related services. It is contemplated by the inventors that the application 860 for implementing exemplary embodiments may be applicable on all commercially available operating systems.

Application 860 may be a source program, executable program (object code), script, or any other entity comprising a set of instructions to be performed. When a source program, then the program is usually translated via a compiler (such as the compiler 840), assembler, interpreter, or the like, which may or may not be included within the memory 820, so as to operate properly in connection with the O/S 850. Furthermore, the application 860 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 870 may include input devices such as, for example but not limited to, a mouse, keyboard, scanner, microphone, camera, etc. Furthermore, the I/O devices 870 may also include output devices, for example but not limited to a printer, display, etc. Finally, the I/O devices 870 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 870 also include components for communicating over various networks, such as the Internet or intranet.

If the computer 800 is a PC, workstation, intelligent device or the like, the software in the memory 820 may further include a basic input output system (BIOS) (omitted for simplicity). The BIOS is a set of essential software routines that initialize and test hardware at startup, start the O/S 850, and support the transfer of data among the hardware devices. The BIOS is stored in some type of read-only-memory, such as ROM, PROM, EPROM, EEPROM or the like, so that the BIOS can be executed when the computer 800 is activated.

When the computer 800 is in operation, the processor 810 is configured to execute software stored within the memory 820, to communicate data to and from the memory 820, and to generally control operations of the computer 800 pursuant to the software. The application 860 and the O/S 850 are read, in whole or in part, by the processor 810, perhaps buffered within the processor 810, and then executed.

When the application 860 is implemented in software it should be noted that the application 860 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 860 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fibre-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibres, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The description has been presented for purposes of illustration and description, and is not intended to be exhaustive or limited to the invention in the form disclosed. Many modifications and variations will be apparent to those of ordinary skill in the art. Embodiments have been chosen and described in order to best explain principles of proposed embodiments, practical application(s), and to enable others of ordinary skill in the art to understand various embodiments with various modifications are contemplated.

## Claims

1. A computer-implemented method for classifying a disease or disability of a subject, the method comprising:
obtaining (110) interaction data associated with a subject, the interaction data being representative of the subject's interaction with a movement-based input device;
processing (120) the interaction data with a first machine learning process to determine a set of characteristics for describing the subject; and
processing (130) the set of characteristics with a second machine-learning process to generate a classification result for the subject, the classification result being representative of a state or progression of a disease or disability of the subject.

2. The method of claim 1, wherein processing (120) the set of characteristics with a second machine-learning process comprises:
comparing the set of characteristics with classification data representing one or more associations between characteristics and disease states; and
based on the result of the comparison, applying a machine-learning based classification process to the set of characteristics.

3. The method of any preceding claim, wherein the classification result for the subject comprises an identification of class or value within a predetermined range of available classes or values for the disease or disability, and optionally wherein the identification comprises a numerical value.

4. The method of any preceding claim, further comprising:
providing (140) an instruction to the subject for directing the subject to interact with the movement-based input device.

5. The method of claim 4, wherein the instruction defines a challenge, and wherein the challenge comprises a time-varying parameter.

6. The method of any preceding claim, wherein at least one of the first machine learning process and second machine learning process employs an artificial neural network.

7. The method of any preceding claim, wherein processing the interaction data with a first machine learning process comprises processing the interaction data with an artificial neural network,
and wherein processing the set of characteristics with a second machine-learning process comprises processing the set of characteristics with an artificial neural network.

8. The method of any preceding claim, further comprising:
training (200) the first machine learning process (220) with interaction data (210) representative of a training subject's interaction with a movement-based input device; and
training (230) the second machine learning process with: classification data (1) representative of a state of a disease or disability of the training subject; and data (z) generated from training the first machine learning process.

9. The method of any preceding claim, further comprising:
assessing at least one of a medication program and treatment program for the subject based on the classification results.

10. A computer program product for classifying a disease or disability of a subject, wherein the computer program product comprises a computer-readable storage medium having computer-readable program code embodied therewith, the computer-readable program code configured to perform all of the steps of any preceding claim.

11. A computer system comprising: a computer program product according to claim 10; and one or more processors adapted to perform a method according to any of claims 1 to 9 by execution of the computer-readable program code of said computer program product.

12. A system (300) for classifying a state of a disease or disability of a subject, the system comprising:
an input interface adapted to obtain interaction data associated with a subject, the interaction data being representative of the subject's interaction with a movement-based input device;
a first machine learning unit (220) adapted to process the interaction data with a first machine learning process to determine a set of characteristics for describing the subject; and
a second machine learning unit (250) adapted to process the set of characteristics with a second machine-learning process to generate a classification result for the subject, the classification result being representative of a state or progression of a disease or disability of the subject.

13. The system of claim 12, further comprising an output interface adapted to provide an instruction to the subject for directing the subject to interact with the movement-based input device.

14. The system of claim 13, wherein the instruction defines a challenge, and wherein the challenge comprises a time-varying parameter.

15. The system of any of claims 12 to 14, further comprising:
an analysis unit adapted to assess at least one of a medication program and treatment program for the subject based on the classification results and to generate an output signal based on the result of the assessment.
